# EUROPEAN PATENT SPECIFICATION

(11) **EP 3 684 391 B1**
(45) Date of publication and mention of the grant of the patent: **02.09.2026**
(21) Application number: 18780248.3
(22) Date of filing: 21.09.2018
(51) Int. Cl.: A61K 36/63, A61P 17/02, A23L 2/00, A61K 31/05

(54) **EXTRACT OF OLIVE VEGETATION WATERS AND/OR OLIVE POMACE FOR USE IN THE REGENERATION OF EPITHELIAL TISSUE**
EIN EXTRAKT AUS OLIVENVEGETATIONSWASSER UND/ODER OLIVENTRESTER ZUR VERWENDUNG IN DER REGENERATION VON EPITHELGEWEBE
EXTRAIT D'EAU DE VÉGÉTATION D'OLIVE ET/OU MARC D'OLIVE POUR L'UTILISATION DANS LA RÉGÉNÉRATION DE TISSU ÉPITHÉLIAL

(30) Priority: 22.09.2017 IT 201700106376
(43) Date of publication of application: 29.07.2020
(73) Proprietor: Fattoria La Vialla Di Gianni, Antonio E Bandino Lo Franco - Societa' Agricola Semplice, 52100 Arezzo (IT)
(72) Inventor: ALBINI, Adriana, 20099 Sesto San Giovanni (Milano) (IT); BRUNO, Antonino, 20156 Milano (IT); DE STEFANO, Daniela, 20128 Milano (IT); TRAMACERE, Matilde Elena, 20133 Milano (IT); NOONAN, Douglas, 20099 Sesto San Giovanni (Milano) (IT)
(74) Representative: Biggi, Cristina
(86) International application number: PCT/IB2018/057301
(87) International publication number: WO 2019/058320

(56) References cited:
- WO-A1-2015/063737
- WO-A1-2016/196950
- WO-A2-03/068171
- SALUCCI SARA ET AL: "Tyrosol prevents apoptosis in irradiated keratinocytes", JOURNAL OF DERMATOLOGICAL SCIENCE, vol. 80, no. 1, 6 July 2015 (2015-07-06), pages 61 - 68, XP029290453, ISSN: 0923-1811, DOI: 10.1016/J.JDERMSCI.2015.07.002

## Description

The present invention relates to the use of a natural phytocomplex rich in polyphenol compounds, in particular rich in hydroxytyrosol and 3,4-DHPA-EDA, derived from the pressing waters of oil olives (commonly known as vegetation waters) and/or pomace from the olive grinding process, in the protection and/or regeneration of body tissues, in particular epithelial tissues.

### PRIOR ART

A characteristic of olive oil that has aroused particular interest has been the high polyphenol content present therein. These compounds are natural antioxidants, of vegetable origin, able to inhibit the formation of free radicals.

The beneficial properties of olive oil have led to a substantial increase, especially in Italy, in the growing of olive trees and the production of oil, with a consequent increase in the production of collateral derivatives of olive oil, mainly vegetation waters and pomace, characterized by a high pollutant load related to a substantial environmental impact.

The disposal of this material is rigorously regulated both at national and regional level and the actuation of the legislation (Law 574 of 11/1996) implies large expense for growers who cannot gain any advantage from these waste products which are however rich in molecules with high medical/pharmaceutical potential.

Hydroxytyrosol constitutes the polyphenol contained in the largest quantity in vegetation waters and represents the most studied compound. It is contained in vegetation waters and in pomace and is also generated by the hydrolysis of oleuropein, a substance contained in particular in the leaves of olive trees.

Recent studies have demonstrated that hydroxytyrosol alone has a cytoprotective effect on PC12 cells (a pheochromocytoma cell line), is anti-apoptotic, when administered to U937 cells (a human myelomonocytic line) and C2C12 (a murine myoblast line), inhibits breast cancer proliferation *in vivo* in the case of induced neoplasia, is chemopreventive in studies on HL60 and HL60R cancer lines (a human promyelocytic leukemia cell line and its multi-drug resistant derivative) and can prevent premenstrual syndrome and osteoporosis.

It has also been demonstrated that the *in vivo* administration of hydroxytyrosol (also at high concentrations, up to 250-500 mg/kg/day) does not have any toxic effect.

Some studies have demonstrated that, when administered alone, oleuropein performs anti-microbial activity, has anti-cancer potential in colorectal cancer cell lines, in metastatic breast cancer and in ER-negative breast cancer cell lines and has the ability to make the architecture of the cell cytoskeleton unstable.

WO 03/068171 discloses the use of olive vegetation water enriched in hydroxytyrosol for the protection of skin and the treatment of sunburns. WO 2016/196950 discloses a pharmaceutical composition comprising endothelial cells and hydroxytyrosol and/or oleuropein for wound healing as well as its effects on keratinocytes, vascularization and reepithelialization.

Although a lot of research has been performed on vegetation waters, there is still a strongly felt need to identify new properties that can give value to these waste products which would otherwise exclusively represent a high cost for the grower and environmental contamination. There is a particularly felt need to identify new nutritional and/or medical-pharmaceutical properties to make use of this waste product.

On this point, the Applicant has surprisingly found that vegetation waters are able to perform a protection and/or regeneration effect on body tissues, in particular epithelial tissues such as, for example, the epithelium of the epidermis or the epithelium coating the mucosae.

In particular, the Applicant has observed that, by concentrating the permeate of the vegetation waters subjected to microfiltration, through reverse osmosis, a phytocomplex is obtained that is rich in polyphenol compounds able to protect and/or regenerate body tissues, in particular epithelial tissues, with greater efficacy with respect to that of pure hydroxytyrosol, i.e. isolated from the vegetation waters and/or pomace through purification techniques.

This effect is particularly advantageous for human health especially in terms of tissue regeneration. In fact, for that purpose the concentrate of vegetation waters of the present invention, alone or in combination with other substances having an emollient, soothing and/or healing action, can be used to prevent tissue injuries, especially affecting the epithelium, e.g. of the epidermis or the epithelium coating the mucosa, and/or for treating such tissues, should they be damaged, by regenerating them.

### BRIEF DESCRIPTION OF THE FIGURES

Further advantages of the present invention will be highlighted in the following detailed description and appended figures, in particular:

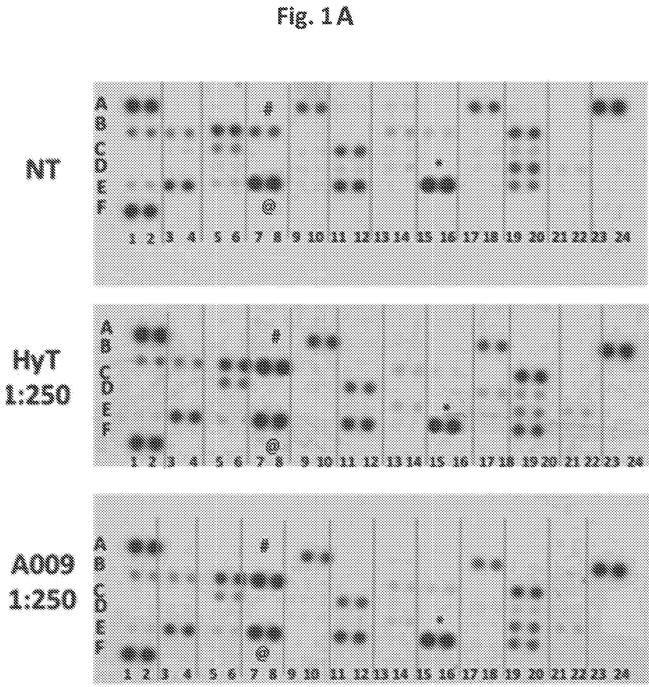

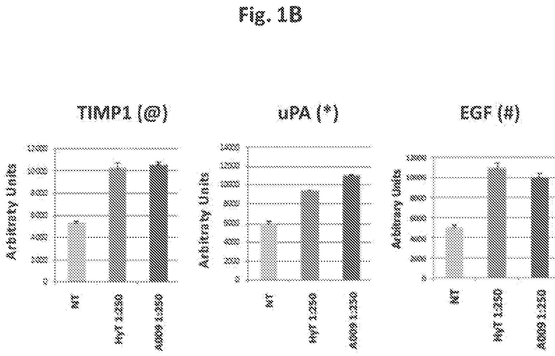

- Figure 1 (A) shows the expression of the TIMP-1 (@), uPA (*) and EGF (#) proteins by an immortalized cell line of epithelial origin, treated with the polyphenol concentrate of the present invention (sample A009) or with purified hydroxytyrosol (HyT), both diluted 1:250 (NT = not-treated cells); the expression of TIMP-1 (@), uPA (*) and EGF (#) proteins has been quantified in arbitrary units and shown in graph (B);

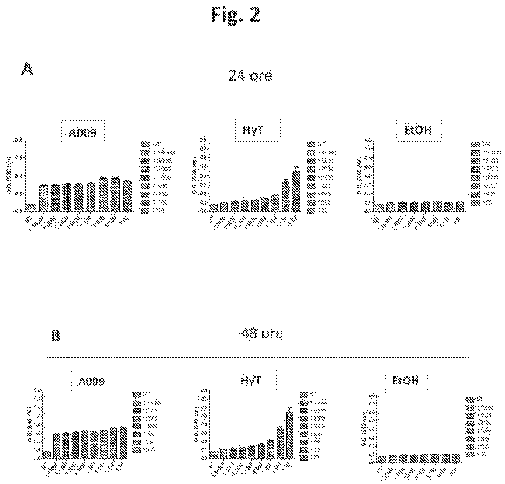

- Figure 2 shows the results of the (3-(4,5-Dimethylthiazol-2-yl)-2,5-Diphenyltetrazolium Bromide (MTT) assay performed to evaluate the proliferation of the cells of the human cell line Ker-CT (ATCC) treated with the polyphenol concentrate of the present invention (sample A009) or with purified hydroxytyrosol (HyT), both at various dilutions. The treatment was performed for 24 hours (A) or for 48 hours (B). NT = not-treated cells; OD = optical density;

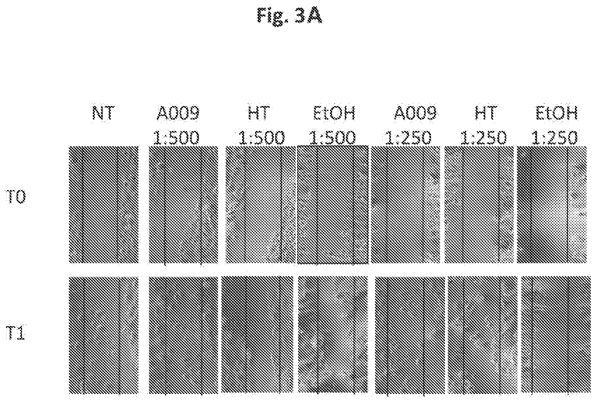

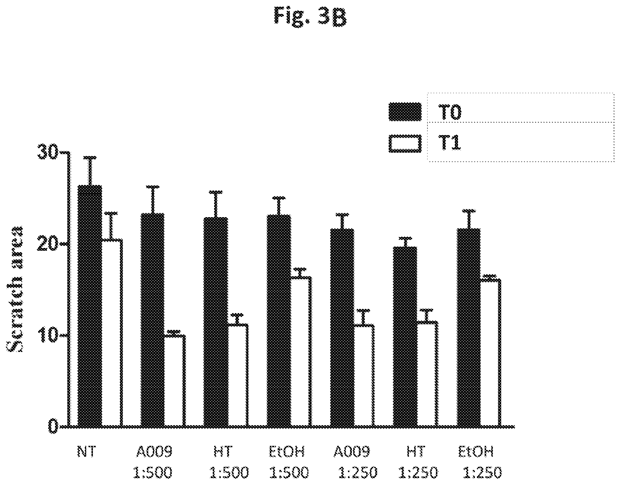

- Figure 3 shows the results of the *in vitro* assay for the evaluation of the tissue regeneration ability (scratch assay) performed on cells of the human cell line Ker-CT treated with the polyphenol concentrate of the present invention (sample A009), with pure hydroxytyrosol (HT), or with ethanol (EtOH, vehicle-control), all diluted 1:500 or 1:250. The images (A) show the tissues injuries (scratches) at the time of their induction (T0) and after 24 hours of treatment (T1). The graph (B) shows the determination of the area between the two edges of the induced injury (scratch area), before and after treatment. NT = not-treated cells; T0 = time at which the tissue injuries were induced (scratches); T1 = after 24 hours of treatment;

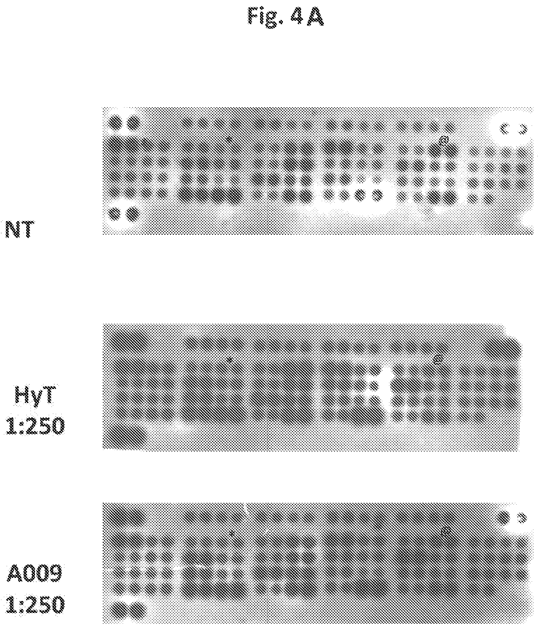

- Figure 4 (A) shows the expression of the EGF (*) and FGF-basic (#) proteins by a Ker-CT human cell line treated with the polyphenol concentrate of the present invention (sample A009) or with pure hydroxytyrosol (HyT) or with ethanol (EtOH, vehicle-control), all diluted 1:500 or 1:250 (NT = not-treated cells); the expression of EGF (*) and FGF-basic (#) proteins has been quantified in arbitrary units and shown in graph (B);

### DETAILED DESCRIPTION OF THE INVENTION

The present invention is defined in the claims.

The present invention relates to a phytocomplex or concentrate of olive vegetation waters and/or pomace comprising the polyphenol compounds hydroxytyrosol and 3,4-DHPA-EDA, for use in the protection and/or regeneration of epithelial tissues, in particular damaged/injured body tissues, said concentrate is obtained by a process comprising the steps of:
(i) microfiltering a sample of the vegetation waters and/or olive pomace so as to obtain a microfiltration concentrate and permeate; and
(ii) concentrating by reverse osmosis the microfiltration permeate of step (i),

. In fact, such concentrate of vegetation waters and/or pomace has shown to be particularly effective in the regeneration of tissue injuries. Reference will be made below to this phytocomplex or concentrate simply with the term "concentrate" or "polyphenol concentrate".

Within the context of the present invention, tissue injury or damage means any type of attack of a physical and/or chemical nature that is able to generate a removal/extirpation of the cell component of the epithelium, epithelium meaning the external coating (skin) and/or internal coating (mucosa). In particular, cutaneous damage/injury means the removal of a portion of cutaneous/epidermal tissue following a physical and/or chemical attack and/or induced by a pathological agent.

Therefore, within the context of the present invention, reepithelialization/healing/regeneration means the process that restores the integrity of the epithelium at the damaged/injured portions/areas, through the re-formation of the epithelium, generally starting from the periphery of the injury/damage/wound and/or from the surviving accessory biological structures such as, for example, in the case of skin, piliferous follicles or sweat glands.

Within the context of the present invention, epithelial tissue means preferably the epidermis or the epithelium coating the mucosae.

The epidermis represents the most superficial (external) portion of the skin. The epithelium of the mucosa covers the internal cavities of the body communicating with the outside, such as those of the digestive apparatus (comprising the oral mucosa), of the respiratory apparatus (comprising the nasal mucosa), of the urinary apparatus and of the genital apparatus.

In a preferred embodiment of the invention, the concentrate is used for the regeneration/protection of injured tissues preferably due to wounds, sores, ulcers, burns, grazes and abrasions. In fact, it has been surprisingly observed that the concentrate described herein highlights healing properties (it can be used as a healing agent) and is therefore, preferably advantageous and effective in the treatment of injuries of the skin and/or the mucosae.

In general, the concentrate of vegetation waters and/or pomace can be used for treating injuries that require the healing and/or reepithelialization and/or regeneration of the epithelium. Vegetation waters preferably derive from an olive grinding process with three phases (oil, vegetation waters and pomace) and/or two phases (oil and pomace + vegetation waters).

The vegetation waters generated by the oil mill can be treated with a solution with acidic pH that preferably varies between 3 and 5, more preferably the pH is about 4/5. The pH is preferably optimized by adding a strong acid and/or pectolytic enzymes, i.e. enzymes that hydrolyze the cellulosic matrix of olive skin.

According to a preferred embodiment of the invention, the pomace is stoned, diluted and/or prefiltered. The pomace preferably has a size that ranges from 0.5 to 1 millimeter (mm), more preferably of about 0.7 mm. An example of the size is that obtained with vibrating screen type sieving. Possibly the stoned pomace is dissolved and/or dispersed in an aqueous matrix with pH preferably comprised between 3 and 5, more preferably between 3.5 and 4.

The dissolving step has the aim of dissolving the polyphenols that would otherwise remain trapped in the solid matrix of the olive skins.

In a preferred embodiment of the invention, the concentrate further comprises: at least one further phenol compound preferably selected from: tyrosol, chlorogenic acid, β-hydroxyverbascoside, rutin, verbascoside, and luteolin; and/or at least one metal preferably selected from: sodium, calcium, magnesium and potassium; and/or at least an anion preferably selected from: chlorides, sulphates, phosphates and nitrates; and/or at least one carbohydrate selected from: glucose, fructose, mannitol and sucrose.

In a further embodiment of the invention the concentrate comprises nitrogenous substances (proteins, aminoacids), preferably in an amount comprised between 15 and 60 mg/kg, more preferably between 20 and 40 mg/kg (mg of nitrogen per liter of active solution).

In any case the phenol compounds contained in the highest amount in the concentrate are hydroxytyrosol and 3,4-DHPA-EDA.

Preferably the amount of hydroxytyrosol ranges between 1 and 10 grams per liter of vegetation waters (g/L), more preferably between 1.5 and 5 g/L, even more preferably between 2 and 3 g/L.

Preferably the amount of 3,4-DHPA-EDA is comprised between 0.5 and 8 g/L, more preferably between 1 and 6 g/L, even more preferably between 1.5 and 2.5 g/L.

Preferably the amount of tyrosol is comprised between 0.1 and 0.4 g/L, more preferably between 0.15 g/L and 0.25 g/L.

Preferably the amount of chlorogenic acid is comprised between 0.06 and 0.24 g/L, more preferably between 0.8 and 0.16 g/L.

Preferably the amount of β-hydroxyverbascoside is comprised between 0.3 and 1.5, more preferably between 0.5 and 1 g/L.

Preferably the amount of rutin is comprised between 0.05 and 0.2 g/L, more preferably between 0.08 and 0.15 g/L.

Preferably the amount of verbascoside is comprised between 0.4 and 1.7 g/L, more preferably between 0.6 and 1 g/L.

Preferably the amount of luteolin is comprised between 0.1 and 0.5 g/L, more preferably between 0.15 and 0.28 g/L.

Preferably the amount of sodium is comprised between 75 and 300 mg/L, more preferably between 120 and 180 mg/L.

Preferably the amount of calcium is comprised between 5 and 10 g/L, more preferably between 2 and 5 g/L.

Preferably the amount of magnesium is comprised between 220 and 900 mg/L, more preferably between 400 and 500 mg/L.

Preferably the amount of potassium is comprised between 3 and 15 g/L, more preferably between 6 and 9 g/L.

Preferably the amount of chlorides is comprised between 1.5 and 7 g/L, more preferably between 2.5 and 4.5 g/L.

Preferably the amount of sulphates is comprised between 12 and 45 g/L, more preferably between 18 and 28 g/L.

Preferably the amount of phosphates is comprised between 1.5 and 7 g/L, more preferably between 2.5 and 5 g/L.

Preferably the amount of nitrates is comprised between 12 and 50 mg/L, more preferably between 18 and 30 mg/L.

Preferably the amount of glucose is comprised between 15 and 60 g/L, more preferably between 25 and 35 g/L.

Preferably the amount of fructose is comprised between 3.5 and 15 g/L, more preferably between 5 and 9 g/L.

Preferably the amount of mannitol is comprised between 1 and 4 g/L, more preferably between 1.5 and 3 g/L.

Preferably the amount of sucrose is comprised between 4 and 16 g/L, more preferably between 6 and 10 g/L.

In the present invention, the concentrate is obtained/obtainable through a process comprising the steps of:
(i) microfiltering a sample of the vegetation waters and/or olive pomace so as to obtain a microfiltration concentrate and permeate; and
(ii) concentrating by reverse osmosis the microfiltration permeate obtained in step (i).

According to a preferred aspect of the invention, microfiltration is performed after the dissolving step as described above.

Microfiltration has the purpose of separating a concentrate, i.e. the concentrated fraction of the suspended content of the vegetation waters/pomace, e.g. micro-fragments, fibers and corpuscular material such as cells and bacteria. It is performed under standard conditions for this type of matrix.

As well as the concentrate, following the microfiltration step a permeate is obtained, i.e. a clear fraction, characterized by a color that varies according to the starting material and that contains the dissolved components of the vegetation waters/pomace, e.g. proteins, sugars, salts, polyphenols, organic acids and various soluble organic molecules. Preferably the microfiltration is performed with at least one, preferably two, ceramic membrane(s). The membrane is characterized by a preferably tubular shape. In a preferred embodiment the membrane is made of aluminum oxide and zirconia.

According to a preferred aspect of the invention, the membrane has the following characteristics:
- an outer diameter that ranges from about 30 to about 40 mm, preferably about 25 mm; and/or
- a length that ranges from about 500 to about 1500 mm, preferably about 1200 mm; and/or
- a series of channels with diameter, preferably hydraulic, that ranges from about 2.5 to about 5 mm, preferably about 3.5 mm; and/or
- a filtering surface area that ranges from about 0.15 to about 0.7 m², preferably about 0.35 m²; and/or
- a molecular size that ranges from about 0.1 micron to about 300 kDa.

In particular, when the membrane is made of ceramic material it is extremely resistant to high temperatures and/or extreme pH conditions and therefore is particularly suitable for the vegetation water treatment process which, producing a high degree of "dirtying" on the membrane, implies washes at high temperatures and also severe pH conditions (e.g. pH13-14).

Furthermore, when the membrane has a tubular shape it allows back pulse washing which is a further system for reconditioning and long-term operation.

The reverse osmosis step, for concentrating the permeate obtained from the microfiltration of the vegetation waters/pomace as described above, is performed under standard conditions for this type of matrix, preferably through the use of a polymeric membrane, more preferably polyamide. Preferably, the membrane has a wound spiral shape and/or a molecular size with high salt rejection, i.e. able to reject the sodium chloride molecule with a percentage of 99.9%. This means that the osmosis membrane retains the molecules of biomedical interest and only lets through the water molecule.

Preferably, the polymeric membrane has a filtering surface area that ranges from about 5 to about 15 m², preferably about 7 m².

The reverse osmosis step allows the permeate obtained from microfiltration to be concentrated preferably about 4 times, which means that from 100 L of microfiltration permeate, 25 L of concentrate are obtained. In this case the volume concentration ratio (VCR) is 4 i.e. 100/25.

The VCR can change based on the starting matrix (vegetation waters) and especially based on its salt content as the reverse osmosis process must counterbalance the osmotic pressure of the matrix that is to be concentrated.

Further subject matter of the present disclosure is constituted by a concentrate (or phytocomplex) of vegetation waters/pomace obtainable/obtained with the process described above.

Preferably said concentrate comprises the substances specified above in the relative amounts, in particular it is hydroxytyrosol and 3,4-DHPA-EDA, where the amount of 3,4-DHPA-EDA is preferably comprised between 0.5 and 8 g/L, more preferably between 1 and 6 g/L, even more preferably between 1.5 and 2.5 g/L and/or the amount of tyrosol is preferably comprised between 0.1 and 0.4 g/L, more preferably between 0.15 g/L and 0.25 g/L. In other words, the concentrate preferably has the composition described above in relation to the content of phenol compounds, and/or metals and/or carbohydrates, and/or anions and/or nitrogen.

A further aspect of the present disclosure relates to a pharmaceutical composition comprising said concentrate and further excipients/pharmacologically accepted ingredients.

According to a further aspect of the disclosure, said concentrate and/or composition is/are used, alone or in combination with other substances, compounds, drugs or compositions with a tissue healing, and/or re-epithelizing, and/or regenerating action, in the protection and/or regeneration of body tissues, in particular of the epithelium and preferably of the epidermis.

Therefore, the concentrate can be applied for preventive purposes to prevent injuries/damage/wounds and/or for curative purposes for treating injuries/damage/wounds already affecting the epithelium.

In one embodiment, the treatment of the epidermis is of particular interest. Therefore, according to the disclosure, the concentrate is used to protect the skin and/or to regenerate it in the event that is it injured/damaged, preferably said injury/damage is associated with/caused by wounds, sores, ulcers, burns, grazes or abrasions.

In another embodiment, the treatment of the epithelium coating the mucosae is of particular interest. Therefore, the concentrate and/or composition are preferably used to protect, or to regenerate the mucosa, in the event of injury/damage. Preferably the mucosa is selected from: the mucosa of the gastrointestinal apparatus, preferably the oral mucosa, the esophageal mucosa, the gastric mucosa and/or the intestinal mucosa; the mucosa of the respiratory apparatus, preferably the nasal mucosa and/or the bronchial mucosa, the mucosa of the urinary apparatus, preferably the urethral and/or vesical mucosa, and the mucosa of the genital apparatus, preferably the uterine mucosa and/or the vaginal mucosa.

In fact, the aforementioned mucosa can be injured/damaged because of wounds, sores, ulcers, burns, grazes or abrasions.

In another preferred aspect of the present invention, the concentrate of vegetation waters and/or pomace and/or the composition as described above is/are in the form of a drink. The drink can further comprise one or more excipients normally contained in the formulation of various types of drinks. The drink enriched with the concentrate of vegetation waters and/or pomace and/or the composition as described above can be defined as functional, i.e. to be used as a food supplement because of the therapeutic effects found and described herein.

Preferably, the drink may be water-based and/or fruit-based and/or milk-based. In a particularly preferred embodiment of the invention, the drink is fruit-based, preferably based on grapes. In particular, grape juice and/or must is preferred, preferably of organic grapes.

Alternatively, the concentrate of vegetation waters and/or pomace described herein and/or the composition can be prepared in the form of oral formulations of various kinds, such as sweets, pills or tablets for oral use.

Also in this case, as for the drink, the oral formulation is taken as a food supplement for the purpose of protecting and/or regenerating body tissues, preferably the epithelium, in particular the epidermis and the epithelium coating the mucosae.

Possibly the drink and/or the oral formulation is/are taken in combination with one or more further substances, compounds, drugs or compositions with a healing action and/or re-epithelizing, and/or regenerating action.

Alternatively, the concentrate of vegetation waters and/or pomace described herein and/or the composition is formulated preferably for topical applications as a cream, oil, ointment, aerosol, shampoo, detergent, gel, ovule or mouthwash. Also in this case the aim is to apply the product for protecting and/or regenerating the epidermal tissue or the epithelium coating the mucosae, through a topical action.

According to a particularly preferred embodiment, the concentrate and/or composition according to the present invention are applied on a support, preferably a plaster, a bandage, a gauze, a spray or a solution.

The concentrate and/or composition can also be in the form of powder, granules, for example following a drying and/or freeze drying process. Said support and/or powder is used for the purpose of healing and/or curing and/or assisting with the wound healing/curing/mending process. The concentrate and/or composition on said support and/or powder may comprise further agents/molecules characterized by a biologically significant function for the purpose of the healing/curing/mending of the wounds, preferably said agents/molecules have anti-inflammatory, antibiotic, healing, emollient, moistening and elasticizing properties.

### EXAMPLES

### Evaluation of the production of epidermal cell proliferation factors following treatment with the concentrate according to the invention.

During a pilot experiment, an immortalized cell line of epithelial origin (HT-29, colorectal adenocarcinoma) was treated with the polyphenol concentrate of the present invention (sample A009) or with hydroxytyrosol (HyT).

With reference to Fig. 1A-B, it is noted that following this treatment a modulation was found in the expression of some factors: in particular an increase in the expression (up-regulation) of the TIMP-1 (@), uPA (*) and EGF (#) proteins was observed, factors involved in the remodelling of tissue following injury.

Tissue Inhibitor of MetalloProteinases 1 (TIMP-1) is a molecule that regulates Matrix MetalloProteinases (MMPs) and Disintegrin-Metalloproteinases (ADAMs and ADAMTSs) by inhibiting them. This makes TIMP-1 particularly significant in regulating the composition of the extracellular matrix also in physiological (such as pregnancy) or pathological (such as the healing of wounds) situations.

Urokinasis, also known as Urokinase-type Plasminogen Activator (uPA) is a serine protease whose primary substrate is represented by plasminogen, the inactive form of serine protease plasmin. The activation of the plasmin performed by uPA triggers a proteolytic cascade which, according to physiological conditions, plays a role in the thrombolysis and/or degradation of the extracellular matrix.

Epidermal Growth Factor (EGF) is a growth factor that stimulates cell survival, the proliferation and differentiation of epidermal cells, through the bond to the respective receptor (EGFR).

The results of the experiment indicate that the concentrate can have an effect on the regeneration of the tissues, in particular on the regeneration of the epidermis, as the cell line used in the experiment is of epithelial origin.

This use of the polyphenol concentrate was also evaluated on a primary line of human keratinocytes.

### Evaluation of the effect of the polyphenol concentrate on the cell proliferation of human keratinocytes.

The effect of the polyphenol concentrate of the invention (A009) on the viability and proliferation of human keratinocytes was evaluated through an MTT colorimetric viability assay (tetrazolium salt, [3-(4,5-dimethylthiazol-2-yl)]-2,5-diphenyltetrazolium bromide).

The MTT assay is based on the ability of the MTT compound to be metabolized by the mitochondrial enzyme succinate dehydrogenase. The reduction of the salt leads to the formation of crystals of a blue product, formazan, which is water-insoluble. Viable cells, unlike non-viable ones, reduce the salt and the amount of formazan produced is proportional to the number of cells contained herein. The crystals formed are dissolved and the absorbance values (or optical density, OD) are measured by reading the spectrophotometer.

The cell module used is a cell line of primary human keratinocytes (Ker-CT) that represent the most common cells contained in the epidermis and that have an important role in cell renewal.

Primary human keratinocytes are placed in culture and treated either with the polyphenol concentrate of the invention (A009), or with hydroxytyrosol (HyT), or with ethanol (EtOH, control); each substance was evaluated at the following dilutions: 1:10000 - 1:5000 - 1:2500 - 1:1000 - 1:500 - 1:250 - 1:100, 1:50; NT indicates absence of cell treatment.

The assays were performed after 24 hours of treatment (Fig.2A) and after 48 hours of treatment (Fig.2B).

The cell viability (and consequently proliferation) was evaluated in terms of optical density measured through the reading on the spectrophotometer at 540 nm, i.e. the optimal wavelength for evaluating cell proliferation according to the number of cells grown and the pH of the growth/culture medium used (7.1-7.2).

With reference to the data presented in Fig.2A-B, an increase in the viability of the primary human keratinocytes treated with the concentrate A009 starting from very high dilutions (1:10000) is observed, already after 24 hours of treatment. The cell proliferation only increases slightly as the concentration of the concentrate increases (i.e. lower dilution), indicating that the efficacy of the concentrate is already maximum at low concentrations.

Hydroxytyrosol (HyT) also stimulates the proliferation of the primary human cells, with an effect that depends on the concentration and that becomes sensitive starting from 1:250 dilution after 24 hours of treatment. The treatment of cells with ethanol, used as a control, does not produce any effect on the cell viability.

The data presented therefore lead to the conclusion that the sample A009 has a more positive effect on (i.e. stimulates) the viability of the primary human keratinocytes with respect to the effect of hydroxytyrosol.

Evaluation of the effect of the polyphenol concentrate on the regeneration of wounds *in vitro.*

The ability of the A009 extract to heal wounds was evaluated through the "scratch assay" method *in vitro.* The cell model used is the primary human keratinocytes (Ker-CT) that are contained in large amounts in the epidermis and that have an important role in skin renewal.

The Ker-CT cells were grown *in vitro* on a solid support until confluence. To perform the scratch assay, a scratch was induced on the cell monolayer using a tip, thus imitating a wound on the epidermis in which the two edges are distanced from each other.

The human primary keratinocytes were then treated with the polyphenol concentrate A009, or with hydroxytyrosol (HyT), or with ethanol (EtOH, control); each substance was evaluated at the dilutions 1:500 and 1:250; NT indicates absence of cell treatment.

After 24 hours of treatment (T1), the treated scratches were photographed with an inverted microscope (Fig.3A) and the regenerative ability was determined, using the analysis software ImageJ, as the area comprised between the edges of the scratch (Fig.3B).

The results obtained and shown in Fig.3A-B demonstrate that the concentrate A009 is able to induce the regeneration of a wound *in vitro* with greater efficacy with respect to that shown by hydroxytyrosol, both at 1:500 dilution and 1:250 dilution.

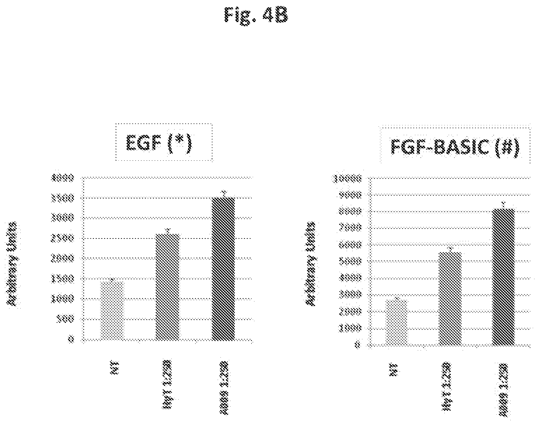

Furthermore, with reference to Fig.4, it has been observed that the concentrate A009 is able to induce the expression of proteins involved in cell remodelling and regeneration, such as EGF and basic FGF.

## Claims

1. Concentrate of olive vegetation waters and/or olive pomace comprising hydroxytyrosol and 3,4-DHPA-EDA or a composition comprising said concentrate, wherein said concentrate is obtained by a process comprising the steps of:
(i) microfiltering a sample of the vegetation waters and/or olive pomace so as to obtain a microfiltration concentrate and permeate; and
(ii) concentrating by reverse osmosis the microfiltration permeate of step (i),
for use in the treatment of a damage or injury of an epithelial tissue, wherein said damage or injury is selected from: wound, sore, ulcer, burn, graze and abrasion.

2. The concentrate or the composition for use according to claim 1, wherein 3,4-DHPA-EDA is in an amount comprised between 0,5 and 8 g/L, preferably between 1 and 6 g/L, more preferably between 1,5 and 2,5 g/L, and/or tyrosol is in an amount comprised between 0,1 and 0,4 g/L, preferably between 0,15 g/L and 0,25 g/L.

3. The concentrate or the composition for use according to claim 1 or 2, wherein said concentrate further comprises:
- at least one phenolic compound preferably selected from: tyrosol, chlorogenic acid, β-hydroxyverbascoside, rutin, verbascoside, and luteolin; and/or
- at least one metal preferably selected from: sodium, calcium, magnesium and potassium; and/or
- at least an anion preferably selected from: chlorides, sulphates, phosphates and nitrates; and/or
- at least one carbohydrate selected from: glucose, fructose, mannitol and sucrose; and/or
- nitrogen.

4. The concentrate or the composition for use according to any one of claims 1-3, wherein the microfiltration step involves the use of at least one ceramic membrane, preferably **characterized by** a tubular shape, more preferably made by aluminum oxide and zirconia.

5. The concentrate or the composition for use according to any one of claims 1-3, wherein the reverse osmosis is performed by using a polymeric membrane, preferably made of polyamide, said membranes being preferably **characterized by** a spiral shape.

6. The concentrate or the composition for use according to any of claims 1-5, wherein said epithelial tissue is the epidermis and/or the mucosal lining epithelium.

7. The concentrate or the composition for use according to any one of claims 1-6, wherein said concentrate and/or composition is in the form of a drink, preferably water and/or fruit and/or milk-based, preferably based on juice and/or grape must.

8. The concentrate or the composition for use according to any one of claims 1-7, wherein said concentrate and/or composition is for topical applications, in the form of a support, preferably a plaster, a bandage or a gauze, and/or in the form of a spray, a solution, a powder or granules.

## Patentansprüche

1. Konzentrat aus Olivenvegetationswasser und/oder Oliventrester, umfassend Hydroxytyrosol und 3,4-DHPA-EDA oder eine Zusammensetzung, umfassend das Konzentrat, wobei das Konzentrat durch ein Verfahren erhalten wird, umfassend die Schritte:
(i) Mikrofiltrieren einer Probe des Vegetationswassers und/oder Oliventrester, um ein Mikrofiltrationskonzentrat und -permeat zu erhalten; und
(ii) Konzentrieren des Mikrofiltrationspermeats von Schritt (i) durch Umkehrosmose,
zur Verwendung bei der Behandlung einer Schädigung oder Verletzung eines Epithelgewebes, wobei die Schädigung oder Verletzung ausgewählt ist aus: Wunde, Übelkeit, Geschwür, Verbrennung, Schürfwunde und Abrieb.

2. Konzentrat oder Zusammensetzung zur Verwendung nach Anspruch 1, wobei 3,4-DHPA-EDA in einer Menge zwischen 0,5 und 8 g/L, vorzugsweise zwischen 1 und 6 g/L, bevorzugter zwischen 1,5 und 2,5 g/L, und/oder Tyrosol in einer Menge zwischen 0,1 und 0,4 g/L, vorzugsweise zwischen 0,15 g/L und 0,25 g/L, vorliegt.

3. Konzentrat oder Zusammensetzung zur Verwendung nach Anspruch 1 oder 2, wobei das Konzentrat ferner Folgendes umfasst:
- mindestens eine phenolische Verbindung, vorzugsweise ausgewählt aus: Tyrosol, Chlorogensäure, β-Hydroxyverbascosid, Rutin, Verbascosid und Luteolin; und/oder
- mindestens ein Metall, vorzugsweise ausgewählt aus: Natrium, Calcium, Magnesium und Kalium; und/oder
- mindestens ein Anion, vorzugsweise ausgewählt aus: Chloriden, Sulfaten, Phosphaten und Nitraten; und/oder
- mindestens ein Kohlenhydrat ausgewählt aus: Glucose, Fructose, Mannitol und Saccharose; und/oder
- Stickstoff.

4. Konzentrat oder Zusammensetzung zur Verwendung nach einem der Ansprüche 1-3, wobei der Mikrofiltrationsschritt die Verwendung mindestens einer keramischen Membran beinhaltet, die vorzugsweise durch eine röhrenförmige Form gekennzeichnet ist, die bevorzugter durch Aluminiumoxid und Zirkoniumdioxid hergestellt ist.

5. Konzentrat oder Zusammensetzung zur Verwendung nach einem der Ansprüche 1-3, wobei die Umkehrosmose unter Verwendung einer polimeren Membran, die vorzugsweise aus Polyamid hergestellt ist, durchgeführt wird, wobei die Membran vorzugsweise durch eine Spiralform gekennzeichnet ist.

6. Konzentrat oder Zusammensetzung zur Verwendung nach einem der Ansprüche 1-5, wobei das Epithelgewebe die Epidermis und/oder das Schleimhautepithel ist.

7. Konzentrat oder Zusammensetzung zur Verwendung nach einem der Ansprüche 1-6, wobei das Konzentrat und/oder die Zusammensetzung in Form eines Getränks, vorzugsweise Wasser und/oder Obst und/oder auf Milchbasis, vorzugsweise auf Saft- und/oder Traubenmostbasis, vorliegt.

8. Konzentrat oder Zusammensetzung zur Verwendung nach einem der Ansprüche 1-7, wobei das Konzentrat und/oder die Zusammensetzung für topische Anwendungen in Form eines Trägers, vorzugsweise eines Pflasters, eines Verbandes oder einer Gaze, und/oder in Form eines Sprays, einer Lösung, eines Pulvers oder Granulats vorliegt.

## Revendications

1. Concentré d'eau de végétation d'olive et/ou marc d'olive comprenant de l'hydroxytyrosol et du 3,4-DHPA-EDA ou une composition comprenant ledit concentré, dans lequel ledit concentré est obtenu par un procédé comprenant les étapes suivantes :
(i) microfiltrer un échantillon d'eau de végétation et/ou marc d'olive de manière à obtenir un concentré et un perméat de microfiltration ; et
(ii) concentrer par osmose inverse le perméat de microfiltration de l'étape (i),
pour l'utilisation dans le traitement d'une lésion ou d'une blessure d'un tissu épithélial, dans lequel ladite lésion ou blessure étant choisie parmi : une plaie, une lésion, un ulcère, une brûlure, une éraflure et une abrasion.

2. Concentré ou composition pour l'utilisation selon la revendication 1, dans lesquels le 3,4-DHPA-EDA est présent dans une quantité comprise entre 0,5 et 8 g/L, de préférence entre 1 et 6 g/L, plus préférablement entre 1,5 et 2,5 g/L, et/ou le tyrosol est présent dans une quantité comprise entre 0,1 et 0,4 g/L, de préférence entre 0,15 g/L et 0,25 g/L.

3. Concentré ou composition pour l'utilisation selon la revendication 1 ou 2, dans lesquels ledit concentré comprend en outre :
- au moins un composé phénolique choisi de préférence parmi : le tyrosol, l'acide chlorogénique, le β-hydroxyverbascoside, la rutine, le verbascoside et la lutéoline ; et/ou
- au moins un métal choisi de préférence parmi : le sodium, le calcium, le magnésium et le potassium ; et/ou
- au moins un anion choisi de préférence parmi : les chlorures, les sulfates, les phosphates et les nitrates ; et/ou
- au moins un glucide choisi parmi : le glucose, le fructose, le mannitol et le saccharose ; et/ou
- l'azote.

4. Concentré ou composition pour l'utilisation selon l'une quelconque des revendications 1-3, dans lesquels l'étape de microfiltration implique l'utilisation d'au moins une membrane céramique, de préférence de forme tubulaire, plus préférablement encore constituée d'oxyde d'aluminium et de zircone.

5. Concentré ou composition pour l'utilisation selon l'une quelconque des revendications 1-3, dans lesquels l'osmose inverse est réalisée à l'aide d'une membrane polymère, de préférence en polyamide, lesdites membranes étant de préférence **caractérisées par** une forme en spirale.

6. Concentré ou composition pour l'utilisation selon l'une quelconque des revendications 1-5, dans lesquels ledit tissu épithélial est l'épiderme et/ou l'épithélium de revêtement muqueux.

7. Concentré ou composition pour l'utilisation selon l'une quelconque des revendications 1-6, dans lesquels ledit concentré et/ou ladite composition se présentent sous la forme d'une boisson, de préférence à base d'eau et/ou de fruits et/ou de lait, de préférence à base de jus et/ou de moût de raisin.

8. Concentré ou composition pour l'utilisation selon l'une quelconque des revendications 1-7, dans lesquels ledit concentré et/ou ladite composition sont destinés à des applications topiques, sous la forme d'un support, de préférence un sparadrap, un bandage ou une gaze, et/ou sous la forme d'un spray, d'une solution, d'une poudre ou de granulés.
